(19) Europäisches Patentamt
European Patent Office
Office européen des brevets

(11) **EP 2 179 719 B1**

(12) **EUROPEAN PATENT SPECIFICATION**

(45) Date of publication and mention
of the grant of the patent:
**12.10.2016 Bulletin 2016/41**

(51) Int Cl.:
*A61K 8/37* *(2006.01)*     *A61Q 13/00* *(2006.01)*
*A61K 8/65* *(2006.01)*     *A61K 8/73* *(2006.01)*
*A61K 8/11* *(2006.01)*     *A61Q 15/00* *(2006.01)*

(21) Application number: **08167669.4**

(22) Date of filing: **27.10.2008**

(54) **Antiperspirant or deodorant compositions**

Schweißhemmende oder desodorierende Zusammensetzungen

Compositions anti-transpirantes ou déodorantes

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**28.04.2010 Bulletin 2010/17**

(73) Proprietors:
• **Unilever PLC**
**London**
**EC4Y 0DY (GB)**
Designated Contracting States:
**CY GB IE MT**
• **UNILEVER N.V.**
**3013 AL Rotterdam (NL)**
Designated Contracting States:
**AT BE BG CH CZ DE DK EE ES FI FR GR HR HU IS IT LI LT LU LV MC NL NO PL PT RO SE SI SK TR**

(72) Inventors:
• **Chan, Catrin Sian**
**Leeds, LS14 2AR (GB)**
• **Cropper, Martin Peter**
**Wirral, Merseyside CH63 3JW (GB)**
• **Franklin, Kevin Ronald**
**Wirral, Merseyside CH63 3JW (GB)**
• **Johnson, Simon Anthony**
**Wirral, Merseyside CH63 3JW (GB)**
• **McKeown, Robert**
**Wirral, Merseyside CH63 3JW (GB)**

(74) Representative: **Whaley, Christopher**
**Unilever Patent Group**
**Colworth House**
**Sharnbrook**
**Bedford MK44 1LQ (GB)**

(56) References cited:
**EP-A- 0 307 723     EP-A- 1 072 259**
**WO-A-2006/056096     WO-A-2007/124889**
**US-B1- 6 171 581**

**Description**

The present invention relates to antiperspirant or deodorant roll-on compositions and in particular to aqueous emulsions. During the course of normal day to day activities, such as from physical activity or from being subjected to stress, mankind perspires, resulting, in the absence of remedial treatment, in damp patches on the skin or in clothing that comes into contact with such skin and in the generation of malodour arising from skin microflora acting upon secretions from inter alia eccrine glands. The distribution of eccrine glands is uneven in the skin, being concentrated particularly in the underarm. Consequently, the underarm is where damp patches and/or malodour generation is most prone to occur.

In many societies, body malodour is considered to be undesirable or even anti-social. Similarly, persons exhibiting damp patches can suffer embarrassment or even criticism. As a consequence, an industry has grown up to prevent or at least hinder sweating and to reduce the generation of body malodour by the creation of cosmetic antiperspirant compositions that are applied topically to skin, particularly in those localised parts of the body where the density of eccrine glands is greatest, such as in the underarm. Whilst bathing can remove sweat, it is not preventative and in any event, there is growing pressure on the world's water so that regular use of antiperspirants as part of a cleanliness regime helps to conserve water supplies. Moreover, the cost of heating water by gas boilers or by electricity generated from gas has increased substantially since 2000, so that regular bathing or showering in an increasingly expensive activity. Commonly, body malodour is counteracted by both washing and regular application of an antiperspirant composition.

The antiperspirant industry has recognised that it is convenient for users to apply an antiperspirant composition after washing, commonly in the bathroom or shower-room such as once or twice a day, for example in the morning or evening, or during preparation for a social gathering. It is less convenient to apply antiperspirant at work or during a social gathering, so that it would be desirable for the effectiveness of an antiperspirant composition to last for several hours, such as up to 24 hours after application.

It would also be reassuring for the user to be reminded periodically of the continuing effectiveness of the antiperspirant composition, removing the anxiety that itself could induce sweating. One of the means adopted by the industry to reduce the impact of malodour generation on the body is the incorporation of a fragrance or a plurality of fragrances into the antiperspirant composition. The fragrance can mask the body odour and some fragrances, so-called deo-fragrances, can also function as a bactericide or bacteristat preventing or retarding the formation of malodorous compounds on the skin.

It has long since been understood that a fragrance functions by being evaporated from the skin onto which it has been applied topically, so that after a while, its concentration falls below that at which it is olfactorily detected. The rate at which that will occur depends on the volatility of the perfume component and its inherent volatility, often approximated by its boiling point. The art of the perfumer is to select a blend of perfume components that satisfies the aesthetic criteria for that perfume. The very fact that the perfume is volatile also means that it can be lost from the composition during storage by preferential evaporation, especially if the container in which the composition is housed is not properly sealed, such as when the cap is not applied properly, such as in liquid contact compositions.

However, it has also been recognised that the perfumer can slow down the rate of evaporation of a perfume by encapsulating it, thereby prolonging the effective life of the perfume after it has been applied topically. Likewise, it was recognised that it was necessary for the perfume to be released from the capsules containing it. In other words, the encapsulation has to be reversible on application, but long lasting during storage prior to application.

The antiperspirant and deodorants industry has developed several different types of composition for applying the antiperspirant or deodorant active material onto the body, including particulate mixtures, anhydrous compositions containing a suspended particulate antiperspirant or deodorant active material and other compositions in which the antiperspirant or deodorant active material is in solution. Each type of composition has its own benefits and its own potential drawbacks or problems, particularly with regard to its manufacture and acceptance by consumers. In fact, some consumers prefer one type of composition and others prefer a different type. So, a solution to a problem in one type of antiperspirant or deodorant composition is often not relevant to the problems with a different type.

One applicator that is popular amongst many users in the world, but especially in Europe, Latin America, South-East Asia, Australasia and Africa is called a roll-on, because it comprises a cylinder or more commonly a ball that rotates within, and partly protruding above, a housing at the open end of a bottle, the surface of the cylinder or ball transporting a film of fluid from within the bottle and enabling it to be rolled onto skin brought into contact with it. Although it is possible to employ anhydrous roll-on formulations based on oils in which an active material is suspended, most roll-on compositions in the last 20 or 30 years have dissolved the active material in a hydrophilic carrier liquid, of which the two most popular carriers have been ethanol (or possibly an alternate short chain aliphatic alcohol, eg up to C6) and water, or aqueous ethanol. Compositions based on the two main sorts of hydrophilic carrier have different benefits and accordingly different advocates.

A number of encapsulating materials have been proposed for encapsulating perfumes, including modified starches and various gums, including acacia gum. Such materials are water soluble at body temperature, so that the disruption of the capsule wall can be triggered by moisture, including, in particular, sweat. Whilst this has the advantage of being localised and triggered by a sweat event, such encapsulates have a significant disadvantage in the context of being incorporated in hydrophilic carrier liquids, namely the extent to which and rate at which they dissolve. The problem of their dissolution in ethanol has been addressed in EP303461 to Unilever, but it is expensive to reduce the water content of ethanol to below about 4% by weight so that substantial leaching of perfume into the carrier occurs during typical transportation and storage periods before the product reaches the shelves of the sales outlet, let alone during the weeks or months after purchase by the consumer. As the proportion of water increases, so the rate and extent of leaching increases greatly. Consequently, such water-sensitive encapsulates are not currently of practical benefit for use in most current roll-on formulations.

It is an object of the present invention in at least some embodiments to ameliorate or overcome one or more of the problems of incorporating fragrances into antiperspirant or deodorant compositions.

It is a further object of some or other embodiments of the present invention to devise aqueous antiperspirant or deodorant compositions that enable triggered release of fragrance over an extended period after application of the composition onto skin.

Brief summary of the present invention

According to one aspect of the present invention, there is provided an antiperspirant or deodorant roll-on composition in the form of an oil-in-water emulsion comprising:-

    a continuous aqueous phase in which is dissolved or dispersed an antiperspirant or deodorant active;
    a dispersed oil phase;
    a nonionic emulsifier or mixture of emulsifiers,
    a dispersed particulate shear sensitive encapsulated perfume, and
    optionally a thickener for the continuous phase in which composition the particulate encapsulated perfume comprises capsules having a shell of a cross-linked gelatin coacervate,
    a volume average particle diameter in the range of 25 to 70 $\mu$m,
    a shell having a measured thickness in the range of from 0.25 to 9 $\mu$m
    a ratio of the shell thickness to the average particle diameter in the range of from 1:5 to 1:120
    and a Hysitron hardness, measured in a Hysitron Tribo-indenter fitted with a Berkovich tip and programmed to perform an indent by compressing a sample with an initial contact force of 75 $\mu$N, for 10 seconds, in the range of from 1.5 MPa to 50 MPa.

By the employment of such a particulate shear sensitive encapsulated perfume satisfying selected characteristics, it is possible to deposit on skin a residual fraction of shear-sensitive encapsulated perfume particles that can be ruptured by the passage of a garment across the surface of the skin or by the movement of one area of skin relative to another, such as in the underarm, at a time when sweating is or is not occurring or irrespective of whether sweating has occurred. Advantage is accordingly taken of the sensitivity of such a capsule on the skin surface to be ruptured by relative movement of garment or skin to skin, but the presence of the thickened or gelled liquid carrier enables the significant fraction of the capsules to be so deposited from conventional contact applicators or from conventional aerosol dispensers. This enables improved masking of malodour and enhanced perception of fragrance over a prolonged period.

Although it is possible for some encapsulates having characteristics outside the ranges identified herein to offer some residual fragrance release activity when freshly incorporated into emulsion compositions, for example encapsulates having a shell formed from starches or related water-soluble or dispersible materials, when triggered by encountering sweat, the selection of encapsulates satisfying the specified ranges according to the present invention continues to provide sustainable benefits even after the composition has been stored for the periods of time commonly needed for transportation of the product to retailers' shelves and whilst in the consumer's possession.

According to a second aspect of the present invention, there is provided the use of a composition according to the first aspect that simultaneously a) prevents or reduces localised sweating by topical application of a composition according to the first aspect and b) prolongs perception of perfume or masks body-generated malodour, even when sweating is not occurring or irrespective of whether sweating has occurred.

The term "emulsion" herein simply requires the oil to be dispersed non-homogeneously within a continuous aqueous phase with emulsifier or emulsifiers present at the interface between the oil and the aqueous phase. It includes compositions in which the oil is present as dispersed droplets.

Detailed Description of the invention, including preferred embodiments.

The present invention relates to the incorporation into emulsion antiperspirant or deodorant roll-on compositions of shear sensitive encapsulated perfume capsules, the term capsules herein including microcapsules. Shear sensitive herein contemplates that the capsule is capable of releasing its perfume contents by rubbing of the upper arm forwards or backwards across in contact with the chest wall whilst remaining in contact with it or by the rubbing of clothing worn on the upper arm or chest likewise rubbing across skin in the upper arm or armpit to which the antiperspirant composition has been applied.

The encapsulating material for the shear-sensitive capsules herein is desirably selected from cross-linked gelatin. One encapsulation process suitable for forming shear sensitive capsules is often called complex coacervation, which has been described, for example, in USP6045835. In such a process, an aqueous solution of a cationic polymer, commonly gelatin or a closely related cationic polymer, is formed at an elevated temperature that is high enough to dissolve the gelatin, commonly at least 40° and in many instances it is unnecessary to exceed 70°C. A range of 40 to 60°C is very convenient. The solution is typically dilute, often falling in the range of from 1 to 10% w/w and particularly from 2 to 5% w/w. Either before or after dissolution of the gelatin, an oil-in-water emulsion is formed by the introduction of a perfume oil, optionally together with a diluent oil if desired.

A polyanion or like negatively charged polymer is introduced and the composition diluted until a pH is attained of below the isoelectric point of the system, such as below pH5, and particular from pH3.5 to pH 4.5, whereupon a complex coacervate forms around the dispersed perfume oil droplets. The polyanion commonly comprises gum arabic or a charged carboxymethyl cellulose derivative, such an alkali metal salt, of which sodium is the most commonly mentioned example.

The resultant shell is subsequently cross linked, with a short chain aliphatic di-aldehyde, for example a $C_4$ to $C_6$ dialdehyde, including in particular glutaraldehyde. The cross linking step is commonly conducted at a temperature of below ambient such as from 5 to 15°C, and particularly in the region of 10°C. Representative weights and proportions of the reactants and of suitable operating conditions are shown in Examples 1, 2 or 3 of the aforementioned US 6045835. The skilled man by suitable selection of the parameters within the general process outlined therein is well capable of producing capsules having a volume average particle size in the range of from 30 to 100μm, particularly up to 75μm and especially 40 to 60μm.

A second encapsulation method that is likewise suitable for forming encapsulated perfumes in which the shell comprises cross-linked coacervated gelatin comprises variations of the above process, as contemplated in WO2006/056096. In such variations, microcapsules comprising a blank hydrogel shell are first formed in a dry state and brought into contact with an aqueous or aqueous/alcoholic mixture of a fragrance compound, commonly diluted with a diluent oil. The fragrance compound is transported through the hydrogel shell by aqueous diffusion and is retained inside. If desired, the resultant fragrance-containing microcapsules can be employed without drying, being a paste or liquid dispersion, or can be dried to a powder, which for practical purposes is anhydrous. Although selection of the ratio of fragrance oil to diluent oil is at the discretion of the producer, and may be varied over a wide range, the ratio is often selected in the range of from 1:2 to 1:1, and particularly 3:4 to 1:1, for fragrance : diluent oils.

The proportion of shell material to core perfume oil is at the discretion of the producer, and is attainable by appropriately varying the proportions of the ingredients in the emulsion. The shell material constitutes 10 to 40% by weight and especially from 12 to 25% by weight of the capsules. By varying the proportions of shell and core, the physical strength of the shell can be varied (for capsules of the same volume average particle size).

Accordingly, capsules having the desired combination of characteristics can be selected.

In some preferred embodiments of the present invention, the fragrance oil constitutes from 70 to 85% by weight of the encapsulates and in such embodiments, the balance is provided by the shell.

In other preferred embodiments, the fragrance oil is present together with an oil diluent, for example providing from 25 to 75% by weight of the oil mixture held within the shell, and especially from 40 to 60% by weight. Desirably in such embodiments, the shell constitutes from 12 to 25% by weight of the encapsulates. In certain of such preferred embodiments, the fragrance constitutes from 35 to 50% by weight of the encapsulates, and is complemented by 35 to 50% by weight of diluent oil. If desired, in yet other embodiments, the composition contains some of the encapsulates that contain diluent oil and others that do not, the weight ratio of the two sets of encapsulates being selected in the range of from 25:1 to 1:25 at the discretion of the producer.

It is preferred for the volume average particle diameter (size) of the capsules to be at least 40 μm and in many desirable embodiments is up to 60 μm in diameter. Herein, unless otherwise indicated, the volume average particle diameter of the encapsulates (D[4,3]) is that obtainable using a Malvern Mastersizer, the encapsulates being dispersed in cyclopentasiloxane (DC245) using a dispersion module mixer speed of 2100 rpm. Calculations are made using the General Purpose model, assuming a spherical particle shape and at Normal calculation sensitivity. The shell thickness can be measured by solidifying a dispersion of the capsules in a translucent oil, cutting a thin slice of the solid mass and using a scanning electron microscope to obtain an image of cut-through individual capsules, thereby revealing the inner and outer outline of its annular shell and hence its thickness.

The shell thickness of the microcapsules tend to increase as the particle size increases. The shell thickness accordingly, often ranges mainly within the thickness range of from 0.25 to 9μm and for many desirable capsules having shells made from coacervated gelatin, at least 90% by volume of the capsules have shells of up to 2.5 μm thickness.

Desirably, at least 95% by volume of the capsules have a shell thickness of at least 0.25 $\mu$m. The average shell thickness of microcapsules desirably employed herein is up to 1.5 $\mu$m. The same or other suitable gelatin coacervate capsules have an average shell thickness of at least 0.4 $\mu$m. For capsules of diameter up to 40 $\mu$m, the shell thickness is often below 0.75 $\mu$m, such as from 0.25 to <0.75$\mu$m whereas for particles of at least 40 $\mu$m the shell thickness is often from 0.6 to 2.5 $\mu$m.

The fragrance-containing capsules for incorporation in the invention antiperspirant compositions are commonly selected having a ratio of volume average diameter:average shell thickness in the range of from 10:1 to 100:1 and in many desirable such capsules in the range of from 30:1 or 40:1 to 80:1.

By virtue of the particle size and the shell thickness of the capsules, the average % volume of the core containing the fragrance oils and any diluent oil, if present, often falls within the range of from 50 to 90%, and in many embodiments from 70 to 87.5%.

The hardness of the capsules, as measured in a Hysitron Tribo-indenter, is an important characteristic that enables them to be incorporated effectively in the invention formulations, retaining the capability of being sheared by frictional contact between skin and skin or clothing. The hardness is desirably in the range of from 0.5 to 50 MPa and especially from 2.5 or 5 up to 25 MPa, and in many embodiments is up to 10 MPa. In certain preferred embodiments, the hardness is in the range of from 3.5 to 5.5 MPa.

A further parameter of interest in relation to the capsules in the instant invention, and particularly their capability to be sheared by friction in the compositions and process of the instant invention, is their "Apparent Reduced Elastic Modulus (Er). Desirably, Er falls within the range of from 20 to 35 MPa, and in many convenient embodiments, in the range of from 22 to 30MPa.

The hardness (Hysitron Hardness) and Apparent Reduced Elastic Modulus herein are those measured by the following method:-

A drop of a dispersion of the capsules in demineralised water is placed onto a piece of silicon wafer and allowed to dry leaving behind discrete micro encapsulates for mechanical analysis. The dried wafer is fitted into the Hysitron Tribo-indenter, and spatially mapped using the optical system of the instrument to identify a perimeter around the sample.

The head of the Tribo-indenter is fitted with a Berkovich tip, a three sided pyramid, which compresses individual capsules. A single capsule is positioned directly under the Indenter tip. The instrument is programmed to perform an indent by compressing the sample with an initial contact force of 75$\mu$N, for 10 seconds, followed by a position hold stage for 1 second and a decompression stage for 10 seconds. The instrument achieves a very small load (typically around 15-30 $\mu$N). The Hysitron Hardness (H in MPa) and reduced Elastic Modulus (Er in MPa) are calculated from the relaxation stage of the force deflection data using the following equations.

$$H = \frac{W}{A}$$

W = Compressive force
A = Contact Area ($A \approx 24.56h_c{}^2$)

$$Er = \frac{\sqrt{\pi}}{2\gamma}\frac{S}{\sqrt{A}}$$

S = Contact Stiffness ($dW/dh_t$)
$h_t$ = Total Penetration Depth
$\gamma$ = 1.034

$$h_c = h_t - \kappa\frac{W}{S}$$

K = 3/4
$h_c$ = Contact Depth

**5**

Results are averages of a minimum of 20 measurements made on capsules with a particle size of D[4,3] +/- 20%

By control of the manufacturing process conditions, the resultant dry capsules having the characteristics specified in the ranges or preferred ranges for particles size and mean diameter described herein can be obtained.

The shear sensitive encapsulate or mixture of encapsulates can be employed in the antiperspirant compositions in an amount at the discretion of the manufacturer. Commonly, the amount is at least 0.05%, in many instances at least 0.1% and often at least 0.3% by weight of the composition. Usually, the amount is up to 5%, desirably up to 4% and in many instances is up to 3% by weight of the composition. A convenient range is from 0.5 to 2.5% by weight of the composition. Accordingly, the base compositions before introduction of propellant contain a proportionately higher proportion of the encapsulate.

The perfume oil employable herein in the shear sensitives capsules, and/or other capsules and/or non-encapsulated can be selected as is conventional to attain the desired aesthetic result, and comprises usually a blend of at least 5 components, and often at least 20 components. The components can be synthetic or natural extractions, and, in the case of natural oils or oils produced to mimic natural oils, are often mixtures of individual perfume compounds. The perfume oil can comprise, inter alia, any compound or mixture of any two or more such compounds coded as an oduor (2) in the Compilation of Odor and Taste Threshold Values Data edited by F A Fazzalari and published by the American Society for Testing and Materials in 1978.

Often, though not exclusively, the perfume compounds acting as perfume components or ingredients in blends have a ClogP (octanol/water partition coefficient) of at least 0.5 and many a ClogP of at least 1. Many of the perfume components that are employable herein can comprise organic compounds having an odour that is discernible by humans that are selected within the chemical classes of aldehydes, ketones, alcohols, esters, terpenes, nitriles and pyrazines. Mixtures of compounds within classes or from more than one class can be blended together to achieve the desired fragrance effect, employing the skill and expertise of the perfumer. As is well known, within the same class, those compounds having a lower molecular weight, often up to about 200, tend to have a lower boiling point and be classified as "top notes", whereas those having a higher molecular weight tend to have a higher boiling point and be classified as middle or base notes. The distinction, though, is to some extent an arbitrary simplification, because the fragrance oils form a continuum and their characteristics are not significantly different close to on either side of an arbitrary boundary such as a boiling point of 250°C or 275°C. Herein, the perfume can comprise any blend of oils boiling at below 250°C (such as in the range 1 to 99% or 4 to 96%, 10 to 90% or 25 to 60%) with the balance provided by compounds having a boiling point above 250°C. The perfumer recognises that the lower boiling point compounds tend to evaporate more quickly after exposure, whereas higher boiling point compounds tend to evaporate more slowly, so that the desired aesthetic effect can be achieved by selecting the proportions of the faster and slower compounds - the faster providing an instant "hit" whilst the slower providing a longer lasting impact. It will also be recognised that a term such as high impact has also been used to describe low boiling point perfume compounds. The properties of the compound stay the same irrespective of whether they are called high impact or top note ingredients.

A further characteristic of a perfume compound is its odour detection threshold (ODT). Some perfume oils are much more easily detected by the human nose than others, but it is a very subjective measurement and varies considerably depending on the way that testing is performed, the prevailing conditions and the make-up of the panel, e.g. age, gender and ethnicity. As a qualitative means of differentiating between the aesthetic attributes of compounds, and enabling the perfumer to choose ingredients that are detected relatively easily, the ODT represents a useful guide, but quantitatively is more dubious.

Examples of perfume compounds or oils that can be employed within the capsules described herein, either singly, or more usually as a blend of at least 5 or at least 10 or at least 20 or at least 50 compounds listed hereinafter include:-1,1,6,7- tetramethyl naphthalene, 1-(2,6,6-trimethyl-1,3-cyclohexandienyl)-2-buten-1-one, 1-(5,5-dimethyl-1-cyclohexen-1-yl)-1h-indene-a-propanal, 1,2,3,4,5,6,7,8-octahydro-1,1,6,7-tetramethyl naphthalene, 1,3-oxathiane, 11-dodecatetraenal, 1-methyl-4-isopropenyl-1-cyclohexene, 2,3-dihydro-1,1-dimethyl-(9ci), 2,4-dimethyl 2,6,-nonenol, 2,4-dimethylcyclohexene-3-carbaldehyde, 2-n-heptyl cyclopentanone, 2,6-nonadienal, 2,6-dimethyl-2,6-octadien-8-ol, 2,6-dimethyl-5-heptenal, 2,6-nonadien-1-ol, 2-isobutylthiazole, 2-methoxy-3- (2-methylpropyl)- pyrazine, 2-methoxy-4-(2-propenyl)phenol, 2-methoxy-4-vinylphenol, 2-methyl-4-propyl-cis-paradiff, 2-propenyl ester, 3- methyl-4-(2,6,6- trimethyl-2- cyclohexen-1-yl) cyclogalbanate, 3-(3-isopropylphenyl)butanal, 3,3-dimethyl-5-(2,2,3-trimethyl-3-cycloenten-1-yl)-4-penten-2-ol, 3,6 nonadienol, 3,7-dimethyl-1,6-octadien-3-ol, 3,7-dimethyl-2, 3,7-dimethyl-2,6-octadienenitrile, 3,7-dimethyl-6-octen-1-ol, 3-buten-2-one, 3-cyclohexadienyl)-3-buten-4-one, 3-cyclohexene-1-carboxaldehyde, 3-methyl-2-buten-1-yl acetate, 3-methyl-4-(2,6,6-trimethyl-2-cyclohexen-1-yl), 3-p-cumenyl-propionaldehyde 4-(1-methylethyl)benzenepropanal, 4 dodecenal, 4-(2,6,6-trimethyl-2-cyclohexen-1-yl)(e), 4-methoxybenzaldehyde, 4-methyl-3-decen-5-ol, 4-methyl-4-mercaptopentan-2-one, 4-penten-1-one, 6-(z,3-pentenyl)-tetrahydro-(2h)-pyranone-2, and 3-methyl-(cis-2-penten-1-yl)-2-cyclopenten-1-one, 6-octadienal, 6-trimethyl-1, 7-acetyl 1 2 3 4 5 6 7 8- octahydro-1, 8-cyclohexadecen-1-one, acetic acid (cyclohexyloxy,2-propenyl ester, acetyl cedrene, acetyl tributly citrateadoxal, aldehyde supra, allyl amyl glycolate, allyl caproate, allyl cyclohexane propionate, allyl heptanoate, allyl heptoate, alpha damascone , alpha methyl ionone iso, alpha pinene, alpha terpineol, alpha thujone, ambrox, ambroxan, amyl acetate, amyl cinnamic aldehyde, amyl salicylate, and isopropyl quinoline, anethol, anisic aldehyde, applinate, aurantiol, bacdanol, benzaldehyde citral polysantol, benzyl acetate, benzyl alcohol, benzyl salicylate, beta gamma hexenol, beta naphthol methyl ether, beta pinene, borneol, bornyl acetate, buccoxime, butyl anthranilate, calone, calone 1951, camphor, carvacrol, carvone, cashmeran, cedramber, cetalox, cinnamic alcohol, cinnamic aldehyde, cis 1,3-oxathiane-2-methyl-4-propyl, cis 3 hexenyl acetate, cis-3-hexenyl acetate, cis-3-hexenyl salicylate, cis-6 nonenol, citral, citronellal nitrile, citronellol, citronellyl acetate, citronellyl oxyacetaldehyde, clonal, corps cassis 0.1% tec, coumarin, cyclal c, cyclemax, cyclo galbanate, cymal, damascene, damascenone, damascone alpha, damascone beta, decyl alcohol, decyl aldehyde, delphone, delta damascone, diacetyl, dihydro iso jasmonate, dihydro myrcenol, dimethyl benzyl carbinol, dimethyl benzyl carbinyl butyrate, dimethyl benzyl carbinyl isobutyrate, d-limonene, ebanol, ethyl 2 4 decadienoate, ethyl 2 methyl butyrate, ethyl aceto acetate, ethyl anthranilate, ethyl butyrate, ethyl caproate, ethyl maltol, ethyl methyl dioxolane acetate, ethyl methyl phenyl glycidate, ethyl vanillin, ethyl-2-4-decadienoate, ethyl-2-methyl- butyrate, eucalyptol, eugenol, exaltolide/cyclopentadecanolide, excital, flor acetate, floralozone, florhydral, fructone, frutene, furaneol, galaxolide, galbex, gamma decalactone, gamma dodecalactone, gamma nonalactone, gamma undecalactone, geraniol, geranyl acetate, geranyl nitrile, geranyl phenylacetate, grapefruit zest (ca), habanolide, helional, heliotropin, heptylcyclopentanone, herbavert, hexahydro-4,7 methano-1h-inden-5(or 6)-yl propionate, hexanoic acid, hexyl acetate, hexyl cinnamic aldehyde, hexyl salicylate, hivemal, hydroquinone dimethyl ether, hydroxycitronellal, indol, intreleven aldehyde, ionone alpha, ionone beta, ionone gamma, irisantheme, iso 2-methoxy-4-(2-propenyl)phenol, isobutyl benzoate, isobutyl cinnamate, iso cyclo citral, iso e super, iso eugenol, iso eugenol acetate, isolongifolanone, iso propyl quinoline, iso-amyl acetate, iso-amyl alcohol, iso methylionone, isononyl acetate, isononyl formate, isovaleric aldehyde 0.1% dpg, javanol, keone, kharismal, labienoxime, lauric aldehyde, lemon juice carbonyls, lilial, liminal, limonene, linalool, linalyl acetate, linalyl benzoate, linalyl cinnamate, lyral, maltol, mandarin aldehyde, manzanate, maple lactone, melonal, menthol, methyl anthranilate, methyl beta naphthyl ketone, methyl cedrylone, methyl cedrylone 0.1041 84.44 811, methyl dihydro jasmonate, methyl dihydrojasmonate, methyl heptine carbonate, methyl isobutenyl tetrahydro pyran, methyl nonyl acetaldehyde, methyl nonyl ketone, methyl octine carbonate, methyl phenyl carbinyl acetate, methyl phenyl carbonyl acetate, methyl-3,4-dioxy(cylcoacetonyl) benzene, nectaryl, neobutanone, nerol, nirvanol, nonalactone, nonanediol-1,3-diacetate, nonanolide-1,4, nonyl aldehyde, nonyl formate, norlimbanol, octyl aldehyde, orange juice carbonyls, orange sinensal, ortho tertiary butyl cyclohexanyl acetate, oxane, oxocyclohexadecen-2-one, ozonil, p.t. bucinal, p-1-menthen-8thiol, para cresol, para cresyl methyl ether, para cymene, para hydroxy phenyl butanone, para hydroxyl phenyl butanone, paradiff, paramenthene, patchouli, pentalide, phenoxy ethyl isobutyrate, phenoxy ethyl propionate, phenyl acetaldehyde, phenyl acetaldehyde dimethyl acetal, phenyl ethyl alcohol, phenyl ethyl dimethyl carbinol, phenyl propyl alcohol, pinoacetaldehyde, polysantol, prenyl acetate, pyrazines, pyrazobutyle, pyridine acetyl 10%, rhubofix, sandalore, sandalore, sandela, spirogalbone, sulfurol, tangerinal, tetra hydro 3,7-dimethyl-1,6-octadien-3-ol, tetrahydrolinalool, tetrahydro muguol, thymol, trans 4 decenal, trans-2-hexenal, tricyclo decenyl acetate, tridecene-2-nitrile, triethyl citrate, trifemal, triplal, undecalactone, undecavertol, undecyl aldehyde, undecylenic aldehyde, vanillin, veloutone, veloutone, verdox, violiff, yara yara, zingerone.

Some of such perfume raw materials have a boiling point of less than, or equal to, 250° C, including some which are generally known to have a low odour detection threshold. Others within said list of perfume raw materials have a boiling point of greater than 250°C of which some are also generally known to have a low odour detection threshold.

Alternatively or additionally, the fragrance incorporated into the capsules can comprise one or a mixture of perfume essential oils, either mixed with each or and/or with synthetic analogues and/or one or more individual perfume compounds, possibly extracted from blossom, leaves, seeds fruit or other plant material. Oils which are herein contemplated include oils from:-

Bergamot, cedar atlas, cedar wood, clove, geranium, guaiacwood, jasmine, lavender, lemongrass, lily of the valley, lime, neroli, musk, orange blossom, patchouli, peach blossom, petotgrain, pimento, rose, rosemary and thyme.

It will be recognised that since naturally derived oils comprise a blend in themselves of many components, and the perfume oil commonly comprises a blend of a plurality of synthetic or natural perfume compounds, the perfume oil itself in the encapsulate does not exhibit a single boiling point, ClogP or ODT, even though each individual compound present therein does.

If desired, the composition can include one or more perfume ingredients that provide an additional function beyond smelling attractively. This additional function can comprise deodorancy. Various essential oils and perfume ingredients, for example those passing a deodorant value test as described in US 4278658 provide deodorancy as well as malodour masking.

The instant invention employs an effective concentration of an antiperspirant or deodorant active, which is say a concentration that is sufficient to reduce or control sweating or reduce or eliminate body malodour. In many desirable embodiments, the composition contains at least 1% antiperspirant active, and preferably at least 5% and often is at least 10%. Commonly, the concentration of the antiperspirant active is not higher than 30%, and in many practical embodiments is not higher than 25.5%, %s herein being by weight based on the composition unless otherwise stated. A preferred concentration range for the antiperspirant active is from 10 to 20%.

The antiperspirant active is conveniently an astringent aluminium and/or zirconium salt, including astringent inorganic salts, astringent salts with organic anions and complexes of such salts. Preferred astringent salts include aluminium, zirconium and aluminium/zirconium halides and halohydrate salts, such as especially chlorohydrates. Activated chlorohydrates can be incorporated, if desired. Some literature employs alternative terminology for chlorohydrates, such as basic aluminium chloride, and aluminium chlorhydrex.

Aluminium halohydrates are usually defined by the general formula $Al_2(OH)_xQ_y.wH_2O$ in which Q represents respectively chlorine, bromine or iodine, (and especially chlorine to form a chlorohydrate) x is variable from 2 to 5 and $x + y = 6$ while $wH_2O$ represents a variable amount of hydration.

Zirconium actives can usually be represented by the empirical general formula: $ZrO(OH)_{2n-nz}B_z.wH_2O$ in which z is a variable in the range of from 0.9 to 2.0 so that the value 2n-nz is zero or positive, n is the valency of B, and B is selected from the group consisting of chlorine (to form a chlorohydrate), other halide, sulphamate, sulphate and mixtures thereof. Possible hydration to a variable extent is represented by $wH_2O$. Preferably, B represents chlorine and the variable z lies in the range from 1.5 to 1.87. In practice, such zirconium salts are usually not employed by themselves, but as a component of a combined aluminium and zirconium-based antiperspirant.

The above aluminium and zirconium salts may have coordinated and/or bound water in various quantities and/or may be present as polymeric species, mixtures or complexes. In particular, zirconium hydroxy salts often represent a range of salts having various amounts of the hydroxy group. Zirconium aluminium chlorohydrate may be particularly preferred.

Antiperspirant complexes based on the above-mentioned astringent aluminium and/or zirconium salts can be employed. The complex often employs a compound with a carboxylate group, and advantageously this is an amino acid. Examples of suitable amino acids include dl-tryptophan, dl-β-phenylalanine, dl-valine, dl-methionine and β-alanine, and preferably glycine which has the formula $CH_2(NH_2)COOH$.

In some compositions, it is highly desirable to employ complexes of a combination of aluminium chlorohydrates and zirconium chlorohydrates together with amino acids such as glycine, which are disclosed in US-A-3792068 (Luedders et al). Certain of those Al/Zr complexes are commonly called ZAG in the literature. ZAG actives generally contain aluminium, zirconium and chloride with an Al/Zr ratio in a range from 2 to 10, especially 2 to 6, an Al/Cl ratio from 2.1 to 0.9 and a variable amount of glycine. Actives of this preferred type are available from Giulini, from Summit and from Reheis.

The invention compositions can comprise, if desired, a deodorant active other than an antiperspirant active described hereinbefore. Such an alternative deodorant active can be selected conveniently from any deodorant active known in the cosmetic art such as antimicrobial actives such as polyhexamethylene biguanides, e.g. those available under the trade name Cosmocil™ or chlorinated aromatics, eg triclosan available under the trade name Irgasan™, nonmicrobiocidal deodorant actives such as triethylcitrate, bactericides and bacteriostatis. Yet other deodorant actives can include bactericidal zinc salts such as zinc ricinoleate. The concentration of such alternative deodorant active is desirably from 0.01 to 5% and in many instances is from 0.1 to 1% by weight of the composition.

In many highly desirable invention compositions, an antiperspirant active is present, either without or supplemented by the alternative deodorant active.

High desirably, the invention emulsions either are at least substantially free from a short chain aliphatic monohydric alcohol, conventionally up to C6, and particularly ethanol. By substantially in this context is meant less than 5% by weight of the composition, preferably less than 3% by weight, particularly less than 1% by weight and more particularly less than 0.5% by weight. Especially preferably, said alcohol and particularly ethanol, is totally absent or at worst less than 0.1% by weight is present.

An essential constituent of compositions of the present invention is a non-ionic emulsifier or mixture of emulsifiers forming an emulsifier system. Such an emulsifier system conveniently has a mean HLB value in the region of from about 5 to about 12 and particularly from 6 to about 10. An especially desired mean HLB value is from 7m to 9. Such a mean HLB value can be provided by selecting an emulsifier having such an HLB value, or more preferably by employing a combination of at least two emulsifiers, a first (lower) HLB emulsifier having an HLB value in the range of from 2 to 6.5, such as in particular from 4 to 6 and a second (higher) HLB emulsifier having an HLB value in the range of from about 6.5 to 18 and especially from about 12 to about 18. When a combination of emulsifiers is employed, the average HLB value can be obtained by a weight average of the HLB values of the constituent emulsifiers.

An especially desirable range of emulsifiers comprise a hydrophilic moiety provided by a polyalkylene oxide (polyglycol), and a hydrophobic moiety provided by an aliphatic hydrocarbon, preferably containing at least 10 carbons and commonly linear. The hydrophobic and hydrophilic moieties can be linked via an ester or ether linkage, possibly via an intermediate polyol such as glycerol.

Preferably the hydrophobic aliphatic substituent contains at least 12 carbons, and is derivable from lauryl, palmityl, cetyl, stearyl, olearyl and behenyl alcohol, and especially cetyl, stearyl or a mixture of cetyl and stearyl alcohols or from the corresponding carboxylic acids. It is particularly convenient to employ an emulsifier comprising a polyalkylene oxide ether.

The polyalkylene oxide is often selected from polyethylene oxide and polypropylene oxide or a copolymer of ethylene oxide and comprises a polyethylene oxide. The number of alkylene oxide and especially of ethoxylate units within suitable emulsifiers is often selected within the range of from 2 to 100. Emulsifiers with a mean number of ethoxylate units in the region of 2 can provide a lower HLB value of below 6.5 and those having at least 4 such units a higher HLB value of above 6.5 and especially those containing at least 10 ethoxylate units. A preferred combination comprises a mixture of an ethoxylate containing 2 units and one containing from 10 to 40 units. Particularly conveniently, the combination of emulsifiers comprises steareth-2 and a selection from steareth-15 to steareth-30.

It is desirable to employ a mixture of ethoxylated alcohol emulsifiers in a weight ratio of emulsifier having a lower HLB value of <6.5 to emulsifier having a higher HLB value of >8 of from 1.5:1 to 6:1 and particularly from 2:1 to 5:1.

The total proportion of emulsifiers in the composition is usually at least 1.5% and particularly at least 2% by weight. Commonly the emulsifiers are not present at above 6%, often not more than 5% by weight and in many preferred embodiments up to 4% by weight. An especially desirable concentration range for the emulsifiers is from 2.5 to 4% by weight.

Another essential constituent of the present invention compositions is an oil, by which is meant a liquid that is water-immiscible. Such oils are characterised by being liquid at 20°C (at 1 atmosphere pressure) and are often selected from silicone oils, hydrocarbon oils, ester oils, ether oils and alcohol oils or a mixture of two or more oils selected from such classes of oils. It is highly desirable that the oil has a boiling point of above 100°C and preferably above 150°C.

The oil is advantageously a plant oil and particularly is a triglyceride oil. Such oils are often obtainable by extraction from the plant's seeds. Suitable plant oils include sunflower seed oil, maize corn oil, evening primrose oil, coriander seed oil, safflower oil, olive oil, rape seed oil, castor oil and borage seed oil. It is particularly desirable to employ an oil which comprises mono or polyunsaturated long chain aliphatic carboxylate substituents, such as notably C18 carboxylates containing 1, 2 or 3 degrees of unsaturation, 2 or more of which may be conjugated. Other suitable oils which come into consideration include jojoba oil.

Alternatively or additionally, the oil can comprise a volatile silicone oil, viz., a liquid polyorgano-siloxane having a measurable vapour pressure at 25°C of at least 1 Pa, and typically in a range of from 1 or 10 Pa to 2kPa. Volatile polyorganosiloxanes can be linear or cyclic or mixtures thereof. Preferred cyclic siloxanes, otherwise often referred to as cyclomethicones, include cyclopenta-methicone and hexacyclomethicone, and mixtures thereof.

The ester oil can be aliphatic or aromatic, commonly containing at least one residue containing from 10 to 26 carbon atoms. Examples of suitable aliphatic oils include isopropyl myristate, isopropyl palmitate, myristyl myristate. Preferably the aromatic ester oil is a benzoate ester. Preferred benzoate esters satisfy the formula Ph-CO-O-R in which R is an aliphatic group containing at least 8 carbons, and particularly from 10 to 20 carbons such as from 12 to 15, including a mixture thereof.

The ether oil preferably comprises a short chain alkyl ether of a polypropylene glygol (PPG), the alkyl group comprising from C2 to C6, and especially C4 and the PPG moiety comprising from 10 to 20 and particularly 14 to 18 propylene glycol units. An especially preferred ether oil bears the INCI name PPG14-butyl ether.

Examples of suitable non-volatile hydrocarbon oils include polyisobutene and hydrogenated polydecene. Examples of suitable non-volatile silicone oils include dimethicones and linear alkylarylsiloxanes. The dimethicones typically have an intermediate chain length, such as from 20 to 100 silicon atoms. The alkylarylsiloxanes are particularly those containing from 2 to 4 silicon atoms and at least one phenyl substituent per silicon atom, or at least one diphenylene group. The aliphatic alcohol desirably is a branched chain monohydric alcohol containing from 12 to 40 carbon atoms, and often from 14 to 30 carbon atoms such as isostearyl alcohol.

Even though, some fragrance oils include an ester group or an ether group, herein the weight of fragrance materials is not included herein in calculating the weight of the oil blend, irrespective of whether the fragrance is encapsulated or "free" (non-encapsulated). The weight of fragrance materials is not included herein in calculating the weight of the oil blend, irrespective of whether the fragrance is encapsulated or "free".

The proportion of oil in the composition (excluding any contribution from water-insoluble constituents of fragrance oils which may be present) is often at least 1% and commonly a least 1.5% by weight. In many instances the proportion of oil is not more than 10% by weight and notably is not more than 5% by weight.

In many suitable embodiments the total proportion of emulsifier(s) and oils (excluding fragrance oils) is selected in the range of from 4 to 7.5% by weight of the emulsion.

The combination of emulsifiers at suitably chosen concentrations can in many embodiments generate a suitable viscosity to enable the composition to function effectively in a roll-on dispenser. However, if desired, a water-soluble or water-dispersible thickener and/or a particulate insoluble thickener can be employed, to increase the viscosity of the composition, thereby enabling a lower overall concentration of emulsifiers to be employed. Such thickeners include water-soluble or water-dispersible polymers include cellulose derivatives such as starches, carboxymethyl cellulose, ethylcellulose polymers, hydroxyethylcellulose polymers, and cellulose ether polymers, and/or gelatins and/or plant-extract polysaccharides thickeners such as extracts from seaweed. Other effective polymeric thickeners include polyethylene oxide, typically with a molecular weight of at least 100,000, and polyacrylic acid. Particulate thickeners include silicas, optionally surface modified, and clays, such as montmorillonite, bentonite and hectorite. The particulate thickeners are finely divided, commonly having a particle size of below 100$\mu$m. Sufficient of such thickener or thickeners is employed to increase the viscosity of the roll-on composition to the desired value.

A preferred constituent of the composition comprises a particulate silica such as an amorphous silica, eg a fumed silica. It is particularly desirable to employ such a fumed (sometimes called pyrogenic) silica which has been hydrophobically treated. Such materials are commercially available under the name hydrophobic silica. Hydrophobic silicas are obtained by chemically bonding a hydrophobic substituent such as especially a siloxane group onto the surface of the silica, possibly following an intermediate treatment in which the surface of the silica has been rendered hydrophilic. Suitable reactants to generate a hydrophobic substituent include halosilanes and in particular chlorosilanes and methylated silazanes such as hexamethyldisilazane. It is particularly desirable to employ a silica that is capable of thickening an oil such as a plant oil.

Desirably, the silica, such as the fumed silica, and especially the hydrophobic silica has a BET specific surface area of at least 100 m$^2$/g and particularly from 150 to 400 m$^2$/g. The silica comprises very fine particles, fumed silica commonly having a diameter for individual particles of below 40 nm and in many instances at least 99% by weight of below 40 nm. In fumed silica as supplied, some aggregation can occur so that in many embodiments, the supplied silica has an average particle size (diameter) of less than or equal to 1000 nm, preferably less than or equal to 500 nm, i.e. the diameter of the silica particle of average weight. In at least some desirable embodiments, at least 99% by weight of the silica particles, as supplied, are in the range of 10 to 500 nm.

The weight proportion of silica in the formulation is often selected taking into account the desired viscosity of the eventual formulation, together with other attributes such as its effect on the speed of drying of the formulation, its perceived greasiness and/or its perceived stickiness. The weight concentration of silica in the composition is desirably at least 0.2%, often at least 0.3% and in many desirable embodiments is at least 0.5% by weight. Its concentration is commonly not greater than 2%, often not greater than 1.5% and in a number of very desirable formulations is not higher than 1.0%. A preferred weight range of silica concentrations is from 0.6 to 0.8%.

The water content of the composition is commonly selected in the range of from 65 to 93% by weight and often from 70 or 75 to 85% by weight.

The weight ratio of silica to water in the invention emulsions is commonly selected in the range of at least 1:400 up to 1:40, often at least 1:275 and in many instances preferably at least 1:200. It is often convenient to employ a weight ratio of up to 1:75.

In addition to the foregoing essential constituents, it is preferable to include a free fragrance, for example in a proportion of from 0.05 or 0.1 to 4% by weight, and particularly from 0.3 to 2% by weight.

In a number of highly desirable embodiments, the invention compositions comprise, by weight, one or more of:-

from 70 to 85% of water;
from 10 to 20% of an antiperspirant active, such as actives described hereinbefore;
from 2.5 to 4.0% of an ethoxylated ether emulsifier or mixture of emulsifiers, preferably having an HLB value of from 7 to 9;
from 1.5 to 4% by weight of a plant oil, such as an unsaturated fatty acid triglyceride;
from 0.5 to 1.0% of a hydrophobic fumed silica from 0.5 to 2.0% of a coacervated gelatin capsules of fragrance and
from 0.3 to 2% of a free fragrance.

By the selection of the proportions of the above identified constituents within the foregoing disclosed ranges of proportions, it is possible to obtain emulsions having a viscosity which fall within a preferred range of from 1000 to 7000 mPa.s and particularly within 2500 to 5500 mPa.s. Viscosities herein are measured in a Brookfield RVT viscometer equipped with a stirrer TA and Hellipath, rotating at 20 rpm at 25°C unless otherwise stated. Such emulsions demonstrate a particularly desirable combination of product attributes such as a desirable speed of drying compared with emulsions lacking the particulate silica, superior greasiness and avoidance of excessive stickiness on application and a superior retention of fragrance release by rubbing or impact for long periods on the skin after topical application.

Preferably, the emulsion is made by first preparing separate aqueous and oil mixtures which are brought together before shearing. The aqueous phase commonly contains the antiperspirant active. Where a mixed emulsifier system is employed, it is desirable to incorporate any emulsifier having a low HLB value, particularly of <6.5 into the oil phase and an emulsifier having a high HLB value, particularly of >6.5 into the aqueous phase. The temperature of the respective phases can be raised, where necessary, to accelerate dissolution of the emulsifier, for example to above 50°C.

It is highly desirable to incorporate silica and especially hydrophobic silica, with the aqueous phase. It is preferable to incorporate any fragrance last of all and shortly before the entire mixture is sheared, especially when either or both phases have been heated so as to accelerate emulsifier dissolution. Though the habits of users vary, commonly a user applies from about 0.2 to 0.4g of composition to an armpit on each application.

In a further aspect of the present invention, there is provided a method of inhibiting perspiration and/or combating malodour perception comprising applying topically to human skin a composition according to the first aspect of the present invention. Advantageously, the composition is applied to localised areas of the body, such as especially in the underarm, but it also be applied to other occluded body areas, such as at the base of the breasts or on the soles of feet.

The invention composition can also be applied via a wipe or a wrist sweat band. The composition is left in place on the body for an extended period, commonly for a period of up to 24 hours, and in many instances from 5 to 18 hours, as is conventional for antiperspirant compositions and thereafter removed by washing in a conventional manner such as using soap and water or showering using shower gels.

The invention formulations are very suitable for dispensing via a roll-on dispenser, for example any dispenser comprising a bottle having a mouth at one end defining a retaining housing for a rotatable member, commonly a spherical ball or less commonly a cylinder which protrudes above the top wall of the bottle. Examples of suitable dispensers are described in EP1175165 or are invert dispensers such as described in USP6511243 or in WO2006/007987 or in WO2006/007991. The bottle mouth is typically covered by a cap, typically having a screw thread that cooperates with a thread on the housing or in an innovative design by a plurality of staggered bayonet/lug combinations. Although in past times the bottle commonly was made from glass with a thermoplastic housing mounted in the mouth of the bottle, most roll-on dispensers are now made entirely from thermoplastic polymers.

Having summarised the invention and described it in more detail, together with preferences, specific embodiments will now be described more fully by way of example only.

The capsules E1 and E2 employed herein comprised a shell made from a complex coacervate of gelatin with respectively gum Arabic or carboxymethylcellose, cross linked with glutaraldehyde. E1 is in accordance with the process of WO2006/056096 and E2 in accordance with US6045835, in each instance with conditions controlled to obtain the specified shell wall and capsule hardness characteristics identified in Table 1 below.

Table 1

| Characteristic | Capsules E1 | Capsules E2 |
|---|---|---|
| Mean particle size D[4,3] | 48.4 $\mu$m | 50.7 $\mu$m |
| Shell thickness (19 to 38$\mu$m) | 0.3-0.65 $\mu$m | |
| Shell thickness (25 to 35$\mu$m) | | 0.25-0.6 $\mu$m |
| Shell thickness calculated at mean particle size | 1.0-1.6 $\mu$m | 1.4-2.2 $\mu$m |
| DR (11 to 18$\mu$m) | 40:1 - 58:1 | 60:1 - 100:1 |
| DR (calculated shell thickness) | 30:1 - 48:1 | 23:1 - 36:1 |
| Hysitron hardness | 4.05 MPa | 4.88MPa |
| Apparent Reduced Elastic Modulus | 24.1 MPa | 27.5 MPa |
| Wt % oils/fragrance in core | 85/40 | 80/80 |

Mean Particle Size: D[4,3] of the capsules after dispersion in volatile silicone (cyclopentadimethicone) was obtained using a Malvern Mastersizer 2000, the following parameters.

- RI of Dispersant = 1.397
- RI of capsule E1 = 1.430
- RI of capsule E2 = 1.530
- Dispersion module mixer speed = 2100rpm.
- Result calculation model = General purpose.
- Calculation sensitivity = Normal.
- Particle shape = Spherical

Shell Thickness: Measured by SEM on encaps with a particle size specified. For non-spherical encaps, the thickness was measured at or close to the minimum encapsulate diameter. Shell Thickness (Calculated): Calculation assumed that capsules were spherical, with a single core and the shell and core had the same density.
DR is the ratio of av. particle diameter : measured shell thickness.

Example 1

In this Example, the effectiveness of emulsion antiperspirant compositions containing a floral (Bm) non-encapsulated fragrance and either containing or lacking an encapsulated fragrance product E1 or E2 were measured and compared.

The effectiveness was determined in the following test in which 24 - 26 panelists self-applied approximately 0.3g example product to either the left or right armpit and comparison product to the other, with overall left-right randomized balance. The test composition containing encapsulated floral-green fragrances E1 and E2 were compared with control formulations that didn't, as specified in the Tables below.

After application of the antiperspirant formulations, the users put on their normal clothing and the intensity of the odour was assessed at 2 hourly intervals on a scale of perception increasing from 0 to 10. The scores were averaged and that for the non-encapsulated sample deducted from that for the encapsulated sample. Three scores were measured, namely intensity of the fragrance itself, the intensity detected through the clothing and finally the intensity of any malodour. The results are summarized in Table 3.

The tested formulations were as follows:-

Table 2

| Ingredient | % by weight | | |
|---|---|---|---|
| Water | Balance | | |
| ACH (50% w/w solution | 30.0 | | |
| Triglyceride oil | 2.0 | | |
| Steareth-2 | 2.3 | | |
| Steareth-20 | 0.9 | | |
| Hydrophobic silica | 0.7 | | |
| Fragrance | E1 | E2 | Bm |
| amount | 1.5 | 0.7 | 1.0 |

The proportion of fragrance employed from E1 and E2 was approximately the same, at about 0.6%.

Table 3 Results

| Assessment time Hrs) | Difference in Intensity at assessment | | | | | |
|---|---|---|---|---|---|---|
| | Direct | | Through Clothing | | Malodour | |
| | Bm+E1 v Bm | Bm+E2 v Bm | Bm+E1 v Bm | Bm+E2 v Bm | Bm+E1 v Bm | Bm+E2 v Bm |
| 0 | 0.86 | 0.29 | 0.38 | 0.45 | n/d | n/d |
| 2 | 0.71 | 0.43 | 0.53 | 0.55 | -0.09 | -0.05 |
| 4 | 1.06 | 0.42 | 0.90 | 0.50 | -0.19 | -0.05 |
| 6 | 1.19 | 0.62 | 1.20 | 0.55 | | 0.05 |
| 8 | 1.43 | 0.09 | 1.00 | 0.60 | -0.23 | -0.20 |
| 10 | 1.33 | 0.29 | 1.00 | 0.20 | -0.24 | -0.40 |
| 12 | 1.28 | 0.48 | 0.80 | 0.05 | -0.43 | -0.05 |
| 14 | 0.95 | 0 | 0.72 | 0.10 | -0.71 | -0.45 |

Table 3 results show that the presence of the encapsulated fragrance tended to show significant improvement in suppressing malodour after the composition had been applied for many hours.

Example 2

Clinical trials were conducted to demonstrate the difference in malodour suppression between encapsulated (test) and non-encapsulated (control) fragrance applied from a roll-on composition. The formulations employed in Example 2 were the same as those employed in Example 1, except that the non-encapsulated fragrance was a different floral fruity fragrance (Cn).

In this Example, test and control product was applied daily to the underarm of panelists (0.3g +/- 0.03g) and the panelist carried out normal daily activities until after 5 or 24 hours, the effectiveness of the fragrance was assessed by the trained assessor rubbing the underarm gently with latex-gloved fingers (10 strokes). After 2 minutes, the malodour was assessed, but on a scale of from 0 to 5. This was repeated on 4 days, with the panelist instructed not to wash the underarm or apply any other antiperspirant or deodorant during the trial.

Table 4 results

| Fragrance Comparison | Assessment before or after shear | Assessment after application (hours) | Odour Score |
|---|---|---|---|
| Cn+E1 v Cn | Before | 5 | -0.11 |
| Cn+E1 v Cn | After | 5 | -0.10 |
| Cn+E1 v Cn | Before | 24 | -0.14 |
| Cn+E1 v Cn | After | 24 | -0.18 |
| Cn+E2 v Cn | Before | 5 | -0.07 |
| Cn+E2 v Cn | After | 5 | -0.09 |
| Cn+E2 v Cn | Before | 24 | -0.12 |
| Cn+E2 v Cn | After | 24 | -0.22 |

Table 4 confirms that presence of the encapsulated fragrances enabled the composition to control malodour better, even after 24 hours.

Examples 3 to 8

The compositions in these Examples made by the same general method as for Example 1 are summarised in Table 5 below.

Table 5

| Example no | 3 | 4 | 5 | 6 | 7 | 8 |
|---|---|---|---|---|---|---|
| Ingredient | % by weight | | | | | |
| ACH[*1] | 25.0 | 30.0 | 35.0 | 40.0 | 30.0 | 30.0 |
| Steareth-2[*2] | 2.3 | 2.3 | 2.0 | 2.0 | 2.3 | 2.6 |
| Steareth-20[*3] | 0.9 | 0.9 | 0.5 | 0.5 | 0.9 | 0.6 |
| Helianthus Annuus[*4] | 2.0 | | | | 2.0 | 4.0 |
| Alkyl Benzoate[*5] | | 2.0 | | | | |
| PPG butyl ether[*6] | | | 4.0 | | | |
| Cyclomethicone[*7] | | | | 4.0 | | |
| Hydrophobic Silica[*8] | 0.7 | 0.3 | 0.7 | | | |
| Fumed Silica[*9] | | | | | 1.0 | 1.5 |
| E1 | 1.6 | | 1.2 | | 1.5 | |
| E2 | | 1.0 | | 0.8 | | 1.2 |
| Free fragrance | 1.5 | 0.8 | 0.8 | 0.8 | 0.8 | |

(continued)

| Example no | 3 | 4 | 5 | 6 | 7 | 8 |
|---|---|---|---|---|---|---|
| Ingredient | % by weight | | | | | |
| Water | balance to 100% | | | | | |

*1 Aluminium Chlorohydrate (50% w/w aqueous solution - Chlorhydrol™ sol - Reheis
*2 Tego Alkanol S2™ - Degussa
*3 Brij 78™ - Uniquema
*4 high oleic - Henry Lamotte
*5 Finsolv TN - Finetex
*6 Fluid AP - Ucon
*7 Cab-o-sil - Cabot
*8 HDK H30™ - Wacker Chemie

**Claims**

1. An antiperspirant or deodorant roll-on composition in the form of an oil-in-water emulsion comprising:-

   a continuous aqueous phase in which is dissolved or dispersed an antiperspirant or deodorant active;
   a dispersed oil phase;
   a nonionic emulsifier or mixture of emulsifiers a dispersed particulate shear sensitive encapsulated perfume, and optionally a thickener for the continuous phase in which composition the particulate encapsulated perfume comprises capsules having a shell of a cross-linked gelatin coacervate,
   a volume average particle diameter in the range of 25 to 70 $\mu$m,
   a shell having a measured thickness in the range of from 0.25 to 9 $\mu$m and providing from 10 to 40% by weight of the capsules,
   a ratio of the shell thickness to the average particle diameter in the range of from 1:5 to 1:120 and a Hysitron hardness, measured in a Hysitron Tribo-indenter fitted with a Berkovich tip and programmed to perform an indent by compressing a sample with an initial contact force of 75 $\mu$N, for 10 seconds, followed by a position hold stage for 1 second and a decompression stage for 10 seconds, in the range of from 1.5 MPa to 50 MPa.

2. A composition according to claim 1 in which the coacervate is obtained by contacting gelatin with either gum Arabic or a charged carboxymethyl cellulose at a pH of below 5.

3. A composition according to claim 1 or 2 in which the coacervate is cross-linked with glutaraldehyde.

4. A composition according to any either claim in which the capsules have a particle size D[4,3] in the range of from 40 to 60$\mu$m.

5. A composition according to any preceding claim in which the capsules have a measured shell thickness in the range of up to 2.5$\mu$m.

6. A composition according to any preceding claim in which the capsules have an average measured shell thickness in the range of from 0.3 to 0.8$\mu$m.

7. A composition according to any preceding claim in which the capsules have an average particle size:shell thickness ratio in the range of from 40:1 to 80:1.

8. A composition according to any preceding claim in which the capsules have an average core volume of from 35 to 55% by volume.

9. A composition according to any preceding claim in which the capsules have a Hysitron hardness in the range of from 2.5 to 4MPa.

10. A composition according to any preceding claim in which the capsules have an apparent reduced elastic modulus in the range of from 10 to 3MPa, measured in a Hysitron Tribo-indenter fitted with a Berkovich tip and programmed to perform an indent by compressing a sample with an initial contact force of 75 μN, for 10 seconds, followed by a position hold stage for 1 second and a decompression stage for 10 seconds.

11. A composition according to any preceding claim which contains from 0.1 to 4% by weight of the capsules.

12. A composition according to any preceding claim which additionally contains non-encapsulated fragrance.

13. A composition according to any preceding claim in which the emulsifier comprises a mixture of non-ionic emulsifiers, one having an HLB value of from 2 to 6.5 and a second having an HLB value of from 6.5 to 18.

14. A composition according to claim 13 in which the emulsifier comprises a mixture of one emulsifier having an HLB value of <6.5 and the second emulsifier having an HLB value of >8 in a weight ratio of from 2:1 to 5:1.

15. A composition according to any preceding claim in which the emulsifier or mixture of emulsifiers is present in an amount together of from 2.5 to 4% by weight of the composition.

16. A composition according to any preceding claim in which the oil is present in an amount of at least 1.5% by weight of the composition.

17. A composition according to any preceding claim in which the oil is a triglyceride oil.

18. A composition according to any preceding claim in which the total proportion of emulsifiers plus oil is in the range of from 4 to 7.5% by weight of the composition.

19. A composition according to any preceding claim which contains a fumed silica in an amount of at least 0.5% by weight.

20. A composition according to claim 19 in which the fumed silica is hydrophobic and present in an amount of from 0.5 to 2.0% by weight.

21. A composition according to any preceding claim in which the antipersirant active is an aluminium and/or zirconium chlorohydrate, optionally completed.

22. A composition according to any preceding claim which contains less than 0.1% by weight or no ethanol.

23. A composition according to any preceding claim in which the composition contains 70 to 85% by weight of water and 10 to 20% by weight of the antiperspirant active.

24. A process for the manufacture of an antiperspirant or deodorant composition comprising the steps of separately forming an oil phase containing an emulsifier having an HLB value of <6.5 and an aqueous phase containing an antiperspirant or deodorant active and an emulsifier having an HLB value of >6.5 and optionally containing a thickener for the aqueous phase, mixing the two phases together and shearing the resultant mixture to form an emulsion, introducing into the mixture shortly before it is sheared a particulate shear sensitive encapsulated perfume in accordance with any one of claims 1 to 11.

25. A method of inhibiting perspiration and/or combating malodour perception comprising applying topically to human skin a composition according any one of claims 1 to 23.

**Patentansprüche**

1. Schweißhemmende oder desodorierende Roller-Zusammensetzung in Form einer Öl-in-Wasser-Emulsion, umfassend:

   eine kontinuierliche wässrige Phase, in der ein schweißhemmender oder desodorierender Wirkstoff gelöst oder dispergiert ist,
   eine dispergierte Ölphase,
   einen nicht-ionischen Emulgator oder eine Mischung von Emulgatoren,

ein dispergiertes teilchenförmiges scherempfindliches eingekapseltes Parfüm und gegebenenfalls ein Verdickungsmittel für die kontinuierliche Phase,

wobei das teilchenförmige eingekapselte Parfüm in der Zusammensetzung Kapseln umfasst mit einer Schale eines vernetzten Gelatinekoazervats,

einem volumengemittelten Partikeldurchmesser in dem Bereich von 25 bis 70 $\mu$m, einer Schale mit einer gemessenen Stärke in dem Bereich von 0,25 bis 9 $\mu$m und

einem Beitrag von 10 bis 40 Gewichts-% der Kapseln,

einem Verhältnis der Schalenstärke zu dem durchschnittlichen Partikeldurchmesser in dem Bereich von 1:5 bis 1:120

und einer Hysitron-Härte, gemessen in einem Hysitron-Triboindenter, versehen mit einer Berkovich-Spitze und programmiert, um einen Eindruck durch Komprimieren einer Probe mit einer Anfangskontaktkraft von 75 $\mu$N für 10 Sekunden auszuführen, gefolgt von einer Positionshaltestufe für 1 Sekunde und

einer Dekompressionsstufe für 10 Sekunden, in dem Bereich von 1,5 MPa bis 50 MPa.

2. Zusammensetzung nach Anspruch 1, worin das Koazervat durch Inkontaktbringen der Gelatine mit entweder Gummi *arabicum* oder einer geladenen Carboxymethylcellulose bei einem pH von unter 5 erhalten wird.

3. Zusammensetzung nach Anspruch 1 oder 2, worin das Koazervat mit Glutaraldehyd vernetzt ist.

4. Zusammensetzung nach irgendeinem Anspruch, worin die Kapseln eine Partikelgröße D[4,3] in dem Bereich von 40 bis 60 $\mu$m aufweisen.

5. Zusammensetzung nach irgendeinem vorhergehenden Anspruch, worin die Kapseln eine gemessene Schalenstärke in dem Bereich bis zu 2,5 $\mu$m aufweisen.

6. Zusammensetzung nach irgendeinem vorhergehenden Anspruch, worin die Kapseln eine gemessene durchschnittliche Schalenstärke in dem Bereich von 0,3 bis 0,8 $\mu$m aufweisen.

7. Zusammensetzung nach irgendeinem vorhergehenden Anspruch, worin die Kapseln ein durchschnittliches Partikelgröße : Schalenstärke-Verhältnis in dem Bereich von 40:1 bis 80:1 aufweisen.

8. Zusammensetzung nach irgendeinem vorhergehenden Anspruch, worin die Kapseln ein durchschnittliches Kernvolumen von 35 bis 55 Volumen-% aufweisen.

9. Zusammensetzung nach irgendeinem vorhergehenden Anspruch, in welchem die Kapseln eine Hysitron-Härte in dem Bereich von 2,5 bis 4 MPa aufweisen.

10. Zusammensetzung nach irgendeinem vorhergehenden Anspruch, worin die Kapseln einen scheinbar reduzierten Elastizitätsmodul in dem Bereich von 10 bis 3 MPa, gemessen in einem Hysitron-Triboindenter, versehen mit einer Berkovich-Spitze und programmiert, um einen Eindruck durch Komprimieren einer Probe mit einer Anfangskontaktkraft von 75 $\mu$N für 10 Sekunden auszuführen, gefolgt von einer Positionshaltestufe für 1 Sekunde und einer Dekompressionsstufe für 10 Sekunden.

11. Zusammensetzung nach irgendeinem vorhergehenden Anspruch, welche 0,1 bis 4 Gew.-% der Kapseln enthält.

12. Zusammensetzung nach irgendeinem vorhergehenden Anspruch, welche zusätzlich nicht-eingekapselten Duftstoff enthält.

13. Zusammensetzung nach irgendeinem vorhergehenden Anspruch, in der der Emulgator eine Mischung von nicht-ionischen Emulgatoren umfasst, wovon einer einen HLB-Wert von 2 bis 6,5 und ein zweiter einen HLB-Wert von 6,5 bis 18 aufweist.

14. Zusammensetzung nach Anspruch 13, worin der Emulgator eine Mischung eines Emulgators mit einem HLB-Wert von < 6,5 und des zweiten Emulgators mit einem HLB-Wert von > 8 in einem Gewichtsverhältnis von 2:1 bis 5:1 umfasst.

15. Zusammensetzung nach irgendeinem vorhergehenden Anspruch, worin der Emulgator oder die Mischung von Emulgatoren zusammen in einer Menge von 2,5 bis 4 Gew.-% der Zusammensetzung vorliegen.

**16.** Zusammensetzung nach irgendeinem vorhergehenden Anspruch, in welcher das Öl in einer Menge von mindestens 1,5 Gewichts-% der Zusammensetzung vorliegt.

**17.** Zusammensetzung nach irgendeinem vorhergehenden Anspruch, in welcher das Öl ein Triglyceridöl ist.

**18.** Zusammensetzung nach irgendeinem vorhergehenden Anspruch, in welcher der gesamte Anteil der Emulgatoren plus Öl in dem Bereich von 4 bis 7,5 Gewichts-% der Zusammensetzung liegt.

**19.** Zusammensetzung nach irgendeinem vorhergehenden Anspruch, welche ein pyrogenes Siliciumdioxid in einer Menge von mindestens 0,5 Gewichts-% enthält.

**20.** Zusammensetzung nach Anspruch 19, in welcher das pyrogene Siliciumdioxid hydrophob ist und in einer Menge von 0,5 bis 2,0 Gewichts-% vorliegt.

**21.** Zusammensetzung nach irgendeinem vorhergehenden Anspruch, in welcher der schweißhemmende Wirkstoff ein Aluminium- und/oder Zirconium-Chlorhydrat, gegebenenfalls komplexiert, ist.

**22.** Zusammensetzung nach irgendeinem vorhergehenden Anspruch, welche weniger als 0,1 Gewichts-% Ethanol oder kein Ethanol enthält.

**23.** Zusammensetzung nach irgendeinem vorhergehenden Anspruch, worin die Zusammensetzung 70 bis 85 Gewichts-% Wasser und 10 bis 20 Gewichts-% des schweißhemmenden Wirkstoffs enthält.

**24.** Verfahren zur Herstellung einer schweißhemmenden oder desodorierenden Zusammensetzung, umfassend die Schritte des separaten Bildens einer Ölphase, die einen Emulgator mit einem HLB-Wert von < 6,5 enthält, und einer wässrigen Phase, die einen schweißhemmenden oder desodorierenden Wirkstoff und einen Emulgator mit einem HLB-Wert von > 6,5 und gegebenenfalls ein Verdickungsmittel für die wässrige Phase enthält, Vermischen der zwei Phasen und Scheren der erhaltenen Mischung, um eine Emulsion zu bilden, Einbringen in die Mischung, kurz bevor sie geschert wird, eines teilchenförmigen scherempfindlichen eingekapselten Parfüms gemäß irgendeinem der Ansprüche 1 bis 11.

**25.** Verfahren zum Verhindern von Transpiration und/oder zum Entgegenwirken der Wahrnehmung üblen Geruchs, umfassend das topische Auftragen einer Zusammensetzung gemäß irgendeinem der Ansprüche 1 bis 23 auf menschliche Haut.

**Revendications**

**1.** Composition à bille d'antiperspirant ou de déodorant dans la forme d'une émulsion huile-dans-eau comprenant :

  une phase aqueuse continue dans laquelle est dissout ou dispersé un actif d'antiperspirant ou de déodorant ;
  une phase d'huile dispersée ;
  un émulsionnant non ionique ou un mélange d'émulsionnants
  un parfum encapsulé sensible au cisaillement particulaire dispersé, et
  éventuellement un épaississant pour la phase continue
  dans laquelle composition le parfum encapsulé particulaire comprend des capsules présentant une enveloppe de coacervat de gélatine réticulée,
  un diamètre moyen de particule en volume dans l'intervalle de 25 à 70 $\mu$m,
  une enveloppe présentant une épaisseur mesurée dans l'intervalle de 0,25 à 9 $\mu$m et fournissant de 10 à 40 % en masse des capsules,
  un rapport de l'épaisseur d'enveloppe au diamètre moyen de particule dans l'intervalle de 1:5 à 1:120 et une dureté Hysitron, mesurée dans un Tribo-indenter Hysitron équipé d'une extrémité Berkovich et programmé pour réaliser une indentation par compression d'un échantillon avec une force de contact initiale de 75 $\mu$N, pendant 10 secondes,
  suivie par une étape de maintien de position pendant 1 seconde et une étape de décompression pendant 10 secondes, dans l'intervalle de 1,5 MPa à 50 MPa.

**2.** Composition selon la revendication 1, dans laquelle le coacervat est obtenu par mise en contact de gélatine avec soit de la gomme arabique soit de la carboxyméthylcellulose chargée à un pH inférieur à 5.

3. Composition selon la revendication 1 ou 2, dans laquelle le coacervat est réticulé avec du glutaraldéhyde .

4. Composition selon l'une quelconque des revendications précédentes dans laquelle les capsules présentent une taille de particule D[4,3] dans l'intervalle de 40 à 60 μm.

5. Composition selon l'une quelconque des revendications précédentes, dans laquelle les capsules présentent une épaisseur d'enveloppe mesurée dans l'intervalle allant jusqu'à 2,5 μm.

6. Composition selon l'une quelconque des revendications précédentes, dans laquelle les capsules présentent une épaisseur d'enveloppe moyenne mesurée dans l'intervalle de 0,3 à 0,8 μm.

7. Composition selon l'une quelconque des revendications précédentes, dans laquelle les capsules présentent un rapport taille moyenne de particule : épaisseur d'enveloppe dans l'intervalle de 40:1 à 80:1.

8. Composition selon l'une quelconque des revendications précédentes, dans laquelle les capsules présentent un volume moyen de noyau de 35 à 55 % en volume.

9. Composition selon l'une quelconque des revendications précédentes, dans laquelle les capsules présentent une dureté Hysitron dans l'intervalle de 2,5 à 4MPa.

10. Composition selon l'une quelconque des revendications précédentes, dans laquelle les capsules présentent un module élastique réduit apparent dans l'intervalle de 10 à 3MPa, mesuré dans un Tribo-indenter Hysitron équipé d'une extrémité Berkovich et programmé pour réaliser une indentation par compression d'un échantillon avec une force de contact initiale de 75 μN, pendant 10 secondes, suivie par une étape de maintien de position pendant 1 seconde et une étape de décompression pendant 10 secondes.

11. Composition selon l'une quelconque des revendications précédentes qui contient de 0,1 à 4 % en masse des capsules.

12. Composition selon l'une quelconque des revendications précédentes qui contient de plus un parfum non-encapsulé.

13. Composition selon l'une quelconque des revendications précédentes, dans laquelle l'émulsionnant comprend un mélange d'émulsionnants non-ioniques, un présentant une valeur HLB de 2 à 6,5 et un second présentant une valeur HLB de 6,5 à 18.

14. Composition selon la revendication 13, dans laquelle l'émulsionnant comprend un mélange d'un émulsionnant présentant une valeur HLB <6,5 et du second émulsionnant présentant une valeur HLB >8 dans un rapport de masse de 2:1 à 5:1.

15. Composition selon l'une quelconque des revendications précédentes, dans laquelle l'émulsionnant ou le mélange d'émulsionnants est présent dans une quantité de 2,5 à 4 % en masse de la composition.

16. Composition selon l'une quelconque des revendications précédentes, dans laquelle l'huile est présente dans une quantité d'au moins 1,5 % en masse de la composition.

17. Composition selon l'une quelconque des revendications précédentes, dans laquelle l'huile est une huile de triglycéride.

18. Composition selon l'une quelconque des revendications précédentes, dans laquelle la proportion totale des émulsionnants plus huile se trouve dans l'intervalle de 4 à 7,5 % en masse de la composition.

19. Composition selon l'une quelconque des revendications précédentes qui contient une silice de pyrogénation dans une quantité d'au moins 0,5 % en masse.

20. Composition selon la revendication 19, dans laquelle la silice de pyrogénation est hydrophobe et présente dans une quantité de 0,5 à 2,0 % en masse.

**21.** Composition selon l'une quelconque des revendications précédentes, dans laquelle l'actif d'antiperspirant est un chlorhydrate d'aluminium et/ou de zirconium, éventuellement complexé.

**22.** Composition selon l'une quelconque des revendications précédentes qui contient moins de 0,1 % en masse ou pas d'éthanol.

**23.** Composition selon l'une quelconque des revendications précédentes, dans laquelle la composition contient de 70 à 85 % en masse d'eau et de 10 à 20 % en masse de l'actif d'antiperspirant.

**24.** Procédé pour la fabrication d'une composition d'antiperspirant ou de déodorant comprenant les étapes de formation séparée d'une phase d'huile contenant un émulsionnant ayant une valeur HLB <6,5 et une phase aqueuse contenant un actif d'antiperspirant ou de déodorant et un émulsionnant ayant une valeur HLB >6,5 et contenant éventuellement un épaississant pour la phase aqueuse, de mélange des deux phases ensemble et de cisaillement du mélange résultant pour former une émulsion,
d'introduction dans le mélange juste avant qu'il soit cisaillé d'un parfum encapsulé sensible au cisaillement particulaire selon l'une quelconque des revendications 1 à 11.

**25.** Procédé d'inhibition de transpiration et/ou de lutte contre une perception malodorante comprenant l'application topique à de la peau humaine d'une composition selon l'une quelconque des revendications 1 à 23.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 303461 A
- US 6045835 A
- WO 2006056096 A
- US 4278658 A
- US 3792068 A
- EP 1175165 A
- US 6511243 B
- WO 2006007987 A
- WO 2006007991 A

**Non-patent literature cited in the description**

- Compilation of Odor and Taste Threshold Values Data. American Society for Testing and Materials, 1978